# EUROPÄISCHE PATENTSCHRIFT

(11) **EP 1 345 912 B1**
(45) Veröffentlichungstag und Bekanntmachung des Hinweises auf die Patenterteilung: **28.07.2004**
(21) Anmeldenummer: 01994815.7
(22) Anmeldetag: 19.12.2001
(51) Int. Cl.: C07D 251/60, C07D 251/62

(54) **VERFAHREN ZUR HERSTELLUNG VON MELAMIN**
METHOD FOR PRODUCING MELAMINE
PROCEDE DE FABRICATION DE LA MELAMINE

(30) Priorität: 27.12.2000 AT 21472000
(43) Veröffentlichungstag der Anmeldung: 24.09.2003
(73) Patentinhaber: AMI Agrolinz Melamine International GmbH, 4020 Linz (AT)
(72) Erfinder: RUECH, Wolfgang, A-4753 Taiskirchen (AT)
(74) Vertreter: Gross, Felix, Dr.
(86) Internationale Anmeldenummer: PCT/EP2001/015057
(87) Internationale Veröffentlichungsnummer: WO 2002/051818

(56) Entgegenhaltungen:
- WO-A-00/55142
- WO-A-01/25221
- WO-A-97/20826
- WO-A-97/47609
- WO-A-98/54160

## Beschreibung

Die Erfindung betrifft die Herstellung von festem Melamin durch Entspannen von flüssigem Melamin unter Zufuhr von gasförmigem Ammoniak unter annähernd Sattdampfbedingungen und Rückführung des freiwerdenden Ammoniaks in den Hochdruckteil der Melaminanlage.

Bei den Hochdruckverfahren zur Herstellung von Melamin wird bei Drücken von meist etwa 50 - 250 bar eine flüssige Rohmelaminschmelze erhalten, welche anschließend in verschiedener Weise verfestigt wird.
US 3,116,294 beschreibt beispielsweise die Verfestigung der Melaminschmelze durch Quenchen mit Wasser, gemäß US 3 637 686 wird die Melaminschmelze mit flüssigem NH3 gequencht und anschließend aus Wasser auskristallisiert. Im wässrigen Medium entstehen jedoch unerwünschte Nebenprodukte.
US 4,565,867 beschreibt die Verfestigung des Melamins durch Quenchen mit flüssigem NH3, und gemäß WO97/34879 wird die Melaminschmelze durch Quenchen mit überkritischem NH₃ kristallisiert, wobei der Druck beim Quenchen gleich oder niedriger als der Druck im Melaminreaktor ist.
Da beim Quenchen mit NH₃ große Mengen an gasförmigem NH₃ anfallen, welche wiederverwertet werden müssen, ist es notwendig, diese Mengen einerseits möglichst zu minimieren und andererseits Bedingungen zu finden, unter denen die anfallende NH3-Menge problemlos wiederverwertet werden kann. Letzteres ist deswegen schwierig, weil das anfallende NH₃ Gas mehr oder minder große Mengen an gasförmigem Melamin enthält, die die Weiterverarbeitung des NH₃-Gases wesentlich erschweren.

Unerwarteterweise wurde nun gefunden, daß die zum Quenchen der Melaminschmelze nötige NH₃-Menge am geringsten ist und andererseits die Wiederverwertung des bei der Melaminkristallisation freiwerdenden NH₃ Gases problemlos möglich ist, wenn die Melaminschmelze mit gasförmigem NH₃ unter annähernd Sattdampfbedingungen bei einem Druck, der über dem Druck des Hochdruckteils der Melaminanlage liegt, gequencht wird und das dabei entstehende melaminhältige NH₃-Gas ohne Zwischenschritte direkt in den Hochdruckteil der Melaminanlage rückgeführt wird.

Gegenstand der Erfindung ist demnach ein Verfahren zur Herstellung von festem, aus einem Hochdruckprozeß ausgehend von Harnstoff stammenden Melamin, das dadurch gekennzeichnet ist, daß flüssiges, ammoniakhältiges Melamin unter gleichzeitiger Zufuhr von gasförmigem NH₃ bei annähernd Sattdampfbedingungen auf einen Druck, der höher als der Druck des Hochdruckteils der Melaminanlage ist, entspannt und dadurch verfestigt wird und das bei der Entspannung freiwerdende Ammoniak in den Hochdruckteil der Melaminanlage rückgeführt wird.

Eine Hochdruckanlage für die Herstellung von Melamin aus Harnstoff besteht beispielsweise aus folgenden Apparaten:
Im Offgaswäscher wird einerseits der Frischharnstoff durch die im Hochdruckteil der Anlage anfallenden heissen Offgase, hauptsächlich aus NH₃ und CO₂ und geringen Mengen Melamin bestehend, vorgewärmt und anschließend dem Melaminreaktor zugeführt. Andererseits wird das heisse Offgas im Wäscher gereinigt, indem das darin enthaltene Melamin in der Frischharnstoffschmelze absorbiert wird.
Im Melaminreaktor wird aus dem vom Wäscher kommenden Harnstoff und aus zusätzlich zugeführtem gasförmigen NH₃ Melamin in Schmelzeform hergestellt und üblicherweise direkt im Reaktor von den ebenfalls gebildeten Offgasen, welche dem Harnstoffwäscher zugeführt werden, abgetrennt.
Die Melaminschmelze wird anschließend dem CO₂-Stripper zugeführt, in welchem durch Einbringen von fein verteiltem Ammoniak-Gas im Gegenstrom das in der Melaminschmelze gelöste CO₂ entfernt und gemeinsam mit dem zugeführten Ammoniak aus dem Apparat ausgetragen wird. Das Gas wird anschließend entweder dem Harnstoffwäscher und/oder dem Melaminreaktor zugeführt.
Es ist auch möglich, die Melaminschmelze aus dem Melaminreaktor einem zweiten Melaminreaktor zuzuführen, in welchen außerdem frische Harnstoffschmelze sowie Ammoniakgas zur Entfernung des in der Melaminschmelze gelösten CO₂ eingebracht wird. Das Abgas aus dem zweiten Melaminreaktor wird dem Harnstoffwäscher und/oder dem Melaminreaktor zugeführt.

Die Apparate Harnstoffwäscher, Melaminreaktor, zweiter Melaminreaktor und CO₂-Stripper werden meist bei annähernd denselben Drücken betrieben und üblicherweise als Hochdruckteil der Melaminanlage bezeichnet. Nach dem Hochdruckteil der Melaminanlage befindet sich eine Druckerhöhungsstufe, im Anschluß daran einer oder mehrere Apparate, in denen die Melaminschmelze bei einem Druck, der über dem Druck des Hochdruckteils der Melaminanlage liegt, eine bestimmte Zeit verweilt. Dies kann beispielsweise ein Agingbehälter und / oder ein Schmelzekühler sein.

Die Melaminschmelze aus einem der Apparate nach der Druckerhöhungsstufe wird von einem Druck p1 von etwa 100 - 1000 bar in einen Expansionsbehälter entspannt.
Die Melaminschmelze ist vor der Expansion bevorzugt mit Ammoniak gesättigt, vorteilhafterweise enthält sie darüberhinaus noch überschüssiges NH₃.

Die Temperatur der Melaminschmelze vor der Entspannung liegt beispielsweise zwischen etwa 250 und 450 °C, bevorzugt zwischen etwa 280 und 400 °C, besonders bevorzugt zwischen etwa 300 und 380 °C. Dabei liegt die Temperatur der Melaminschmelze möglichst nahe, bevorzugt 1-50°C, besonders bevorzugt 1-20°C über ihrem vom jeweils herrschenden Ammoniakdruck abhängigen Schmelzpunkt.

Die Einbringung der Melaminschmelze in den Expansionsbehälter erfolgt über eine geeignete Sprühvorrichtung wie beispielsweise ein Ventil oder eine Düse.
Gleichzeitig mit der Melaminschmelze wird dem Expansionsbehälter gasförmiges Ammoniak in fein verteilter Form beispielsweise über Düsen, Ejektoren oder Ventile zugeführt. Dabei kann das gasförmige NH₃ separat in den Expansionsbehälter eingebracht werden, oder es wird direkt in der Sprühvorrichtung mit der Melaminschmelze vermischt und gemeinsam mit ihr in den Expansionsbehälter eingedüst.

Der Druck des zugeführten Ammoniaks kann sowohl unterkritisch, das heißt kleiner als 113 bar oder überkritisch, das heißt größer als 113 bar sein, oder er kann genau beim kritischen Druck liegen. In jedem Fall liegt der Ammoniakdruck höher als der Druck im Expansionsbehälter.

Die Temperatur des zugeführten Ammoniaks kann ebenfalls in einem weiten Bereich schwanken. Dabei wird das Ammoniak bei annähernd Sattdampfbedingungen verwendet, das heißt die Temperatur des Ammoniaks liegt in einem Bereich von etwa 0-100 °C, bevorzugt 5-50 °C, besonders bevorzugt 5-20 °C über dem Taupunkt, falls der Druck unterkritisch ist beziehungsweise über der kritischen Temperatur, falls der Druck überkritisch ist. Besonders vorteilhaft ist es, Ammoniak einzusetzen, dessen Temperatur in Abhängigkeit vom jeweils herrschenden Druck möglichst wenig oberhalb des Taupunktes beziehungsweise möglichst wenig oberhalb der kritischen Temperatur liegt. In diesem Fall ist die Wärmekapazität des Ammoniak maximal, sodaß die zur Wärmeabfuhr bei der Melaminverfestigung nötige Ammoniak-Menge minimiert wird.

Die Melaminschmelze wird im Expansionsbehälter vom Druck p1 auf den Druck p2 entspannt. Der Druck p2 ist dabei höher, bevorzugt um etwa 1 - 25 bar höher als der Druck im Hochdruckteil der Melaminanlage. Der Druck im Hochdruckteil der Melaminanlage liegt üblicherweise zwischen etwa 50 und 250 bar. Der Druck p2 liegt vorteilhafterweise zwischen etwa 51 und 275 bar.

Durch den Kontakt der heißen Melaminschmelze mit dem kalten gasförmigen Ammoniak und die Druckerniedrigung von p1 auf p2 im Expansionsbehälter wird die Melaminschmelze verfestigt. Die bei der Melaminkristallisation freiwerdende Kristallisationswärme wird vom Ammoniak abgeführt, das dadurch vorgewärmt wird.

Die Temperatur des Melamins nach der Verfestigung kann gleich oder niedriger als die Temperatur der eingebrachten Melaminschmelze sein.
Die Melaminverfestigung kann unter isothermen Bedingungen durchgeführt werden. Dabei liegt das Melamin nach der Verfestigung bei der gleichen Temperatur wie die dem Expansionsbehälter zugeführte Melaminschmelze vor. In diesem Fall erfolgt die Kristallisation des Melamins ausschließlich durch die Druckerniedrigung von p1 auf p2, und das zugeführte Ammoniak dient zur Aufnahme der freiwerdenden Kristallisationswärme.
Ebenso ist es möglich, daß die Temperatur des Melamins nach der Verfestigung niedriger als die Temperatur der dem Expansionsbehälter zugeführten Melaminschmelze ist. In diesem Fall dient das zugeführte gasförmige NH₃ nicht nur zum Abführen der Kristallisationswärme sondern auch als Kühlmittel für die Melaminschmelze.
Die Temperatur des Melamins nach der Verfestigung liegt in einem Bereich von zwischen etwa 200 °C und dem vom jeweiligen Ammoniakdruck abhängigen Schmelzpunkt des Melamins. Sie liegt beispielsweise zwischen etwa 250 und 350°C, bevorzugt zwischen etwa 280 und 350 °C, besonders bevorzugt zwischen etwa 300 und 350 °C. Dabei soll die Temperatur des Melamins möglichst nahe, beispielsweise 1-100°C, bevorzugt 1-50°C, besonders bevorzugt 1-20°C unter ihrem vom jeweils herrschenden Ammoniakdruck abhängigen Schmelzpunkt liegen. Dadurch werden optimale Bedingungen für ein nachfolgendes Tempern des festen Melamins erreicht.

Das bei der Melaminverfestigung freiwerdende NH₃ kann anschließend direkt in den Hochdruckteil der Melaminanlage rückgeführt werden. Es ist auch möglich, das durch die Melaminverfestigung vorgewärmte NH₃ vor der Einbringung in den Hochdruckteil der Melaminanlage weiter vorzuwärmen, beispielsweise mit Hilfe von Salzschmelze als Wärmeträger.

Die Rückführung des bei der Verfestigung freigewordenen NH₃ kann in verschiedene Apparate des Hochdruckteils erfolgen. Beispielsweise kann das Ammoniak dem Melaminreaktor zugeführt werden. Dem Melaminreaktor wird üblicherweise überschüssiges NH₃ Gas in einer Menge von bis zu 10 mol NH₃ / mol Harnstoff zugeführt, um den Harnstoffumsatz zu verbessern, eine bessere Durchmischung im Reaktor zu gewährleisten oder um Verkrustungen zu vermeiden. Das bei der Verfestigung freigewordene NH₃ kann auch dem zweiten Melaminreaktor, dem CO₂-Stripper oder dem Harnstoffwäscher zugeführt werden. Es ist auch möglich, das gesamte, bei der Verfestigung des Melamins freiwerdende NH₃ auf zwei oder mehrere Apparate des Hochdruckteils der Melaminanlage aufzuteilen. Dabei ist es möglich, einzelne oder alle NH₃-Ströme vor der Einbringung in den jeweiligen Apparat weiter vorzuwärmen.

Das im Expansionsbehälter kristallisierte Melamin kann anschließend einer Tempereinheit zugeführt werden, in welcher das feste Melamin unter möglichst hohem Ammoniakdruck bei einer Temperatur, die möglichst knapp unterhalb des Melaminschmelzpunktes liegt, für eine bestimmte Zeit verweilt.

Anschließend wird die Temperatur des Melamins, beispielsweise in einem Dünnschichtkühler, auf Raumtemperatur erniedrigt, gleichzeitig wird auf Atmosphärendruck entspannt, worauf das feste Melamin isoliert wird.

## Patentansprüche

1. Verfahren zur Herstellung von festem, aus einem Hochdruckprozeß ausgehend von Harnstoff stammenden Melamin, **dadurch gekennzeichnet, daß** flüssiges, ammoniakhältiges Melamin unter gleichzeitiger Zufuhr von gasfömigem Ammoniak bei annähernd Sattdampfbedingungen auf einen Druck, der höher als der Druck des Hochdruckteils der Melaminanlage ist, entspannt und dadurch verfestigt wird und das bei der Entspannung freiwerdende gasförmige Ammoniak in den Hochdruckteil der Melaminanlage rückgeführt wird.

2. Verfahren gemäß Anspruch 1, **dadurch gekennzeichnet, daß** die Temperatur des zugeführten gasförmigen Ammoniak um 0 - 100 °C, bevorzugt um 5 - 50 °C, besonders bevorzugt um 5 - 20 °C über seinem druckabhängigen Taupunkt liegt, falls der Druck des zugeführten Ammoniak unter dem kritischen Druck liegt oder um 0 - 100 °C, bevorzugt um 5 - 50 °C, besonders bevorzugt um 5 - 20 °C über der kritischen Temperatur liegt, falls der Druck des zugeführten Ammoniak über dem kritischen Druck liegt.

3. Verfahren gemäß Anspruch 1, **dadurch gekennzeichnet, daß** die Entspannung von einem Druck p1 von etwa 100 - 1000 bar auf einen Druck p2 von etwa 51 - 275 bar erfolgt, wobei p2 um 1 - 25 bar höher als der Druck im Hochdruckteil der Melaminanlage liegt.

4. Verfahren gemäß Anspruch 1, **dadurch gekennzeichnet, daß** die Temperatur des flüssigen, ammoniakhältigen Melamins vor dem Entspannen um 1 - 50 °C, bevorzugt um 1 - 20 °C über seinem vom jeweiligen Ammoniakdruck abhängigen Schmelzpunkt liegt.

5. Verfahren gemäß Anspruch 1, **dadurch gekennzeichnet, daß** das flüssige, ammoniakhältige Melamin aus einem Agingbehälter oder einem Schmelzekühler stammt.

6. Verfahren gemäß Anspruch 1, **dadurch gekennzeichnet, daß** das flüssige, ammoniakhältige Melamin vor der Entspannung mit Ammoniak gesättigt ist.

7. Verfahren gemäß Anspruch 1, **dadurch gekennzeichnet, daß** die Temperatur des Melamins nach der Verfestigung gleich oder niedriger als die Temperatur des flüssigen, ammoniakhältigen Melamins vor der Entspannung ist.

8. Verfahren gemäß Anspruch 1, **dadurch gekennzeichnet, daß** im Anschluß an die Verfestigung das Melamin einer Temperbehandlung unterzogen wird.

9. Verfahren gemäß Anspruch 1, **dadurch gekennzeichnet, daß** das bei der Entspannung freiwerdende Ammoniak in den Melaminreaktor und/oder in einen zweiten Melaminreaktor und/oder in den CO₂-Stripper und/oder den Harnstoffwäscher rückgeführt wird.

## Claims

1. Process for producing solid melamine coming from a high-pressure process starting from urea, **characterized in that** liquid, ammonia-containing melamine is depressurized with simultaneous introduction of gaseous ammonia under approximately saturated vapour conditions to a pressure which is higher than the pressure of the high-pressure section of the melamine plant and is thereby solidified and the gaseous ammonia liberated in the depressurization is recirculated to the high-pressure section of the melamine plant.

2. Process according to Claim 1, **characterized in that** the temperature of the gaseous ammonia introduced is 0-100°C, preferably 5-50°C, particularly preferably 5-20°C, above its pressure-dependent dew point if the pressure of the ammonia introduced is below the critical pressure or is 0-100°C, preferably 5-50°C, particularly preferably 5-20°C, above the critical temperature if the pressure of the ammonia introduced is above the critical pressure.

3. Process according to Claim 1, **characterized in that** the depressurization occurs from a pressure p1 of about 100-1000 bar to a pressure p2 of about 51-275 bar, with p2 being 1-25 bar higher than the pressure in the high-pressure section of the melamine plant.

4. Process according to Claim 1, **characterized in that** the temperature of the liquid, ammonia-containing melamine prior to depressurization is 1-50°C, preferably 1-20°C, above its melting point at the respective ammonia pressure.

5. Process according to Claim 1, **characterized in that** the liquid, ammonia-containing melamine comes from an ageing container or a melt cooler.

6. Process according to Claim 1, **characterized in that** the liquid ammonia-containing melamine is saturated with ammonia prior to depressurization.

7. Process according to Claim 1, **characterized in that** the temperature of the melamine after solidification is equal to or lower than the temperature of the liquid, ammonia-containing melamine prior to depressurization.

8. Process according to Claim 1, **characterized in that** the melamine is subjected to a heat treatment subsequent to solidification.

9. Process according to Claim 1, **characterized in that** the ammonia liberated in the depressurization is recirculated to the melamine reactor and/or to a second melamine reactor and/or to the CO₂ stripper and/or the urea scrubber.

## Revendications

1. Procédé en vue de la fabrication de mélamine solide provenant d'urée, issue d'un processus haute pression, **caractérisé en ce que** l'on procède à la détente de la mélamine fluide, contenant de l'ammoniac, conjointement à un apport simultané d'ammoniac gazeux dans des conditions s'approchant de la vapeur saturée, à une pression qui est supérieure à la pression de la partie haute pression de l'installation de mélamine, et ainsi à sa solidification et **en ce que** l'ammoniac gazeux, libéré lors de la détente, est reconduit dans la partie haute pression de l'installation de mélamine.

2. Procédé selon la revendication 1, **caractérisé en ce que** la température de l'ammoniac gazeux acheminé se situe autour de 0 - 100°C, de préférence, de 5 - 50°C, en particulier, de préférence, de 5 - 20°C au-dessus de son point de rosée dépendant de la pression, au cas où la pression de l'ammoniac acheminé se situe en dessous de la pression critique ou se situe de 0 - 100°C, de préférence, de 5 - 50°C, en particulier, de préférence, de 5 - 20°C au-dessus de la température critique, au cas où la pression de l'ammoniac acheminé se situe au-dessus de la pression critique.

3. Procédé selon la revendication 1, **caractérisé en ce que** la détente se fait d'une pression p1 d'environ 100 - 1000 bars à une pression p2 d'environ 51 - 275 bars, p2 se situant à un niveau de 1 - 25 bars de plus que la pression dans la partie haute pression de l'installation de mélamine.

4. Procédé selon la revendication 1, **caractérisé en ce que** la température de la mélamine fluide, contenant de l'ammoniac, se situe avant la détente de 1 - 50°C, de préférence, de 1 - 20°C, au-dessus de son point de fusion dépendant de la pression d'ammoniac correspondante.

5. Procédé selon la revendication 1, **caractérisé en ce que** la mélamine fluide, contenant de l'ammoniac, provient d'un récipient de vieillissement ou d'un refroidisseur de masse fondue.

6. Procédé selon la revendication 1, **caractérisé en ce que** la mélamine fluide, contenant de l'ammoniac, est saturée avant la détente à l'aide d'ammoniac.

7. Procédé selon la revendication 1, **caractérisé en ce que** la température de la mélamine après la solidification est égale ou inférieure à la température de la mélamine fluide, contenant de l'ammoniac, avant la détente.

8. Procédé selon la revendication 1, **caractérisé en ce qu'**à la suite de la solidification, on soumet la mélamine à un traitement de recuit.

9. Procédé selon la revendication 1, **caractérisé en ce que** l'ammoniac libéré lors de la détente est reconduit dans le réacteur de mélamine et/ou dans un deuxième réacteur de mélamine et/ou dans le dispositif de stripage de CO₂ et/ou dans le laveur d'urée.
